Europäisches Patentamt

European Patent Office

Office européen des brevets

(19)

(11) Numéro de publication : **0 515 246 A2**

# (12) DEMANDE DE BREVET EUROPEEN

(21) Numéro de dépôt : **92401307.1**

(22) Date de dépôt : **13.05.92**

(51) Int. Cl.⁵ : **A23J 3/10**, A23J 3/00, A23J 3/16, A23J 3/04, A23J 3/26, A23L 1/314

(30) Priorité : **13.05.91 FR 9105757**

(43) Date de publication de la demande :
**25.11.92 Bulletin 92/48**

(84) Etats contractants désignés :
**BE CH DE DK ES FR GB IT LI NL**

(71) Demandeur : **UNIVERSITE MONTPELLIER II SCIENCES ET TECHNIQUES DU LANGUEDOC**
**Place E. Bataillon**
**F-34095 Montpellier Cédex (FR)**

(72) Inventeur : **Queguiner, Claire**
**Les jardins du Père Soulas**
**Bâtiement R 118 34000 Montpellier (FR)**
Inventeur : **Cheftel, Jean-Claude**
**8 rue Coîmbre**
**34000 Montpellier (FR)**
Inventeur : **Dumay, Eliane**
**8 rue Coîmbre**
**34000 Montpellier (FR)**

(74) Mandataire : **Peaucelle, Chantal et al**
**Cabinet Armengaud Aine 3, avenue Bugeaud**
**F-75116 Paris (FR)**

(54) **Compositions protéiques à textures onctueuse.**

(57)    L'invention concerne des compositions protéiques élaborées à partir de protéines globulaires et de caséinates.
    Ces compositions sont utilisables notamment dans la préparation d'analogues de matières grasses et de bases hydratantes.

EP 0 515 246 A2

L'invention a pour objet des compositions protéiques à texture onctueuse.

On sait que des compositions de ce type, en particulier des compositions protéiques laitières, sont utilisées comme substituts de matières grasses afin de remplacer, en partie ou en totalité, les matières grasses de produits alimentaires.

Diverses compositions ont été ainsi proposées pour élaborer des produits dits allégés.

Ainsi, dans la demande de brevet EP 0 250 623 au nom de John Labatt Ltd, on décrit un substitut de matières grasses formé par une composition protéique macrocolloïdale essentiellement constituée de particules non agrégées de protéines de lactosérum, ou de blanc d'oeuf, dénaturées. Le procédé de dénaturation comprend le chauffage des protéines à une température de 80 à 130°C environ, à pH acide, avec application d'intenses forces de cisaillement.

Dans la demande de brevet EP 0 340 035, au nom de la société Kraft Inc., le substitut de matières grasses décrit est préparé à partir de complexes polysaccharide/protéine microfragmentés sous d'intenses forces de cisaillement. Différentes protéines alimentaires sont envisagées telles que les protéines de blanc d'oeuf, de lactosérum, de soja, des caséinates, ou encore du lait écrémé, ainsi que différents polysaccharides comme le xanthane, les carragénates, la carboxyméthyl cellulose, l'alginate ou le chitosane. La microfragmentation des complexes polysaccharide/protéine est réalisée à l'aide de microfluidiseurs, ou d'homogénéiseurs très puissants.

La demande de brevet EP 0345 226 au nom de la société Nutrasweet rapporte l'utilisation de micelles de caséine concentrées et non agrégées comme substitut de matières grasses dans les aliments de pH supérieur à 5,6. Les micelles de caséine sont obtenues préférentiellement par ultrafiltration de lait écrémé.

La demande de brevet international WO 89/05587 au nom de la société Nutrasweet, propose un substitut de matières grasses préparé plus particulièrement à partir de microparticules composées d'un noyau (micelles de caséines, cellulose microcristalline, protéines insolubles), enveloppé d'une couche de protéines coagulées (protéines de blanc d'oeuf, concentré protéique de lactosérum, soja, sérum albumine bovine).

Les inventeurs ont recherché une autre solution pour l'élaboration de compositions protéiques à texture onctueuse de pH proche de la neutralité et donc utilisables sans autre traitement dans des produits alimentaires de pH voisin de la neutralité. Ces travaux ont montré que de telles compositions pouvaient être obtenues à faible coût en ajoutant, à des protéines d'un type donné, des dérivés de caséine.

L'invention a donc pour but de fournir une composition protéique à texture onctueuse, directement utilisable dans des aliments de pH proche de la neutralité.

Elle vise également à fournir un procédé d'obtention de ces compositions exploitable à l'échelle industrielle.

Selon un autre aspect, l'invention vise l'application des compositions protéiques en question dans l'alimentation ou encore comme base en cosmétique ou en pharmacie.

Les compositions protéiques de l'invention sont caractérisées en ce qu'elles sont à base de caséinates et de microparticules coagulées de protéines globulaires.

D'une manière surprenante, l'addition de caséinates aux protéines globulaires empêche les phénomènes d'agrégation observés à pH neutre lorsqu'on soumet par exemple un isolat protéique de lactosérum à un traitement de thermocoagulation.

L'utilisation en combinaison de ces deux types de composés permet de disposer de préparations de texture onctueuse, présentant un goût neutre.

L'homme de l'art ajustera aisément les proportions respectives de ces composés afin d'obtenir la texture recherchée, étant entendu qu'une proportion en protéine globulaire supérieure à environ 60 % en poids provoque une texture granuleuse tandis qu'une proportion inférieure à 30 % peut donner une texture à caractère collant.

Avantageusement les protéines globulaires sont présentes à raison de 40 à 50 % en poids environ, le reste de la composition étant essentiellement constitué par les caséinates. Le pH de ces compositions est proche de la neutralité, plus spécialement de l'ordre de 6 à 7,5 environ, ce qui les rend particulièrement appropriées pour être directement utilisables dans des produits de pH neutre, sans nécessiter d'ajustement de pH.

Dans ces compositions, les microparticules de protéines globulaires coagulées sont dispersées dans une matrice de caséinate.

La taille des microparticules, telle que mesurée par diffractométrie laser, n'excède pas 20 µm environ. Ce résultat est d'autant plus inattendu que la thermocoagulation à pH neutre d'un isolat protéique de lactosérum, même sous régime d'agitation, aboutit à un produit de texture très granuleuse, composé de particules coagulées de 0,5 à 2 mm environ.

Des protéines globulaires particulièrement appropriées pour la mise en oeuvre de l'invention comprennent notamment celles de lactosérum, les protéines de blanc d'oeuf, de soja ou de sérum sanguin.

Les protéines de lactosérum sont sous forme de concentrés ou d'isolats.

Les caséinates sont avantageusement formés par du caséinate de calcium.

Selon un autre aspect, les compositions protéiques de l'invention sont telles qu'obtenues par microcoagulation d'un mélange essentiellement formé de protéines globulaires et de caséinates, en appliquant un traitement thermique et des forces de cisaillement de manière à obtenir une dimension de microparticules de 20 μm environ au plus, tout en conservant les qualités biologiques et la valeur nutritionnelle des produits utilisés.

Dans ces compositions, les protéines globulaires sont partiellement déplissées, agrégées et insolubilisées par le traitement thermomécanique d'extrusion, le caséinate restant soluble.

L'enthalpie résiduelle de dénaturation, exprimée par rapport à un mélange témoin, est de l'ordre de 30 à 40 % lorsqu'on opère à des températures ne dépassant pas 100°C environ.

Cette microcoagulation d'un mélange de protéines de lactosérum et de caséinate en proportions précises est obtenue par association d'un traitement thermique modéré et de forces de cisaillement.

Le procédé selon l'invention d'obtention des compositions protéiques définies ci-dessus est caractérisé en ce qu'on mélange les ingrédients de départ, sous forme pulvérulente, les protéines globulaires étant mises en oeuvre de préférence à raison de 40 à 50 % en poids et qu'on soumet le mélange obtenu à un traitement de cuisson extrusion, à une température de l'ordre de 80 à 120°C, de préférence de 85 à 90°C, en appliquant des forces de cisaillement permettant d'obtenir une fragmentation des protéines coagulées, et des microparticules dont la taille n'excède pas 20 μm environ.

On ajoute de l'eau ou une solution diluée acide ou basique au mélange pulvérulent avant d'effectuer le traitement thermique de coagulation.

La teneur totale en protéines peut varier pendant l'extrusion. Elle est de préférence comprise entre 15 et 25 %, selon la fermeté souhaitée pour le produit final.

Pour la mise en oeuvre de ce procédé, on utilise avantageusement un cuiseur-extrudeur bi-vis à fourreau long, à sections modulaires.

Un profil de vis préféré comprend 3 zones dans lesquelles sont effectués respectivement :
1/ le transport, l'hydratation des matières pulvérulentes et l'homogénéisation du mélange avec l'eau,
2/ la coagulation thermique du mélange sous régime d'agitation, et
3/ le refroidissement partiel.

Le procédé de l'invention présente l'avantage de pouvoir être réalisé en continu et en effectuant séquentiellement ou simultanément plusieurs opérations unitaires.

L'utilisation d'un cuiseur-extrudeur bi-vis à fourreau long et à sections modulaires permet d'ajuster avec précision température, forces de cisaillement et temps de séjour. Enfin, des produits de teneurs en eau et de viscosités très différentes peuvent être traités, tout en assurant un débit très régulier.

D'autres appareils continus ou discontinus permettant un traitement thermomécanique efficace peuvent être également utilisés pour la préparation des compositions protéiques de l'invention, par exemple des échangeurs de chaleur à surface raclée ou encore des mélangeurs homogénéiseurs.

Les mélanges de protéines ainsi préparés, de pH proche de la neutralité, constituent des ingrédients alimentaires (et non des additifs) qui peuvent être utilisés librement, sans aucune autorisation préalable, étant donné leur nature. Par leur texture lisse et onctueuse, leur saveur neutre et leur couleur blanche, ils se prêtent tout particulièrement à être utilisés comme substituts de matières grasses dans des aliments très divers. Facilement dispersibles dans l'eau ou dans divers produits alimentaires, ils engendrent une onctuosité habituellement attribuable aux matières grasses ou aux émulsions huile/eau et eau/huile.

La texture onctueuse des protéines microcoagulées à pH proche de la neutralité est maintenue après acidification jusqu'à pH 5,3. Elles peuvent donc être utilisées dans des aliments de pH supérieur ou égal à 5,3.

L'invention vise donc des produits allégés caractérisés en ce qu'il s'agit de produits alimentaires renfermant en combinaison une composition protéique telle que définie ci-dessus.

La quantité de composition protéique sera ajustée en fonction du produit alimentaire de manière à conserver le goût et à obtenir l'onctuosité souhaitée.

Avantageusement les produits alimentaires contenant ces substituts de matière grasse élaborés à partir des compositions de l'invention, présentent un pH neutre ou légèrement acide.

A titre d'exemple, on mentionnera l'utilisation des compositions protéiques de l'invention dans les produits laitiers, tels que laits, fromages frais, fromages, beurres, pâtes à tartiner, desserts, crèmes, crèmes glacées, crèmes pour nappage, yaourts.

Ces compositions sont également utilisables pour élaborer des produits de même type, mais à base de soja.

Les compositions de l'invention conviennent également comme analogues de matières grasses dans les produits à base d'huile, tels que sauces pour salades, mayonnaises, ou margarines.

De même, elles sont utilisables dans des pâtes à tartiner non laitières, des pâtisseries et des plats cuisinés apertisés, congelés ou conservés congelés, ou encore pour préparer de la charcuterie, notamment des préparations carnées émulsifiées.

Après introduction à des doses variables dans divers aliments, les compositions protéiques de l'invention peuvent être soumises à un traitement thermique, par exemple de pasteurisation. Ce chauffage doit de préférence être effectué à une température inférieure ou égale à celle mise en oeuvre pendant la microcoagulation par cuisson-extrusion. En cas de traitement thermique à une température supérieure, la texture des protéines microcoagulées à pH proche de la neutralité risque de devenir plus granuleuse. Cependant cette évolution est moins marquée 1) lorsque les protéines microcoagulées sont introduites dans les aliments à faible dose et 2) lorsque des inhibiteurs d'agrégation protéique sont ajoutés.

Les composés inhibiteurs d'agrégation pouvant être introduits lors de la microcoagulation du mélange protéique comprennent des polysaccharides chargés comme le xanthane, les pectines, les carrhagénates, ou les alginates, des maltodextrines, des polysaccharides neutres comme les amidons solubles, hydrolysés ou modifiés, des mono ou disaccharides (0,1 à 1 %, p/p), ou des agents émulsifiants tels que le stearoyl lactylate de sodium ou de calcium (0,1 à 1 %, p/p). L'introduction de ces composés permet de faire subir sans dommages un traitement thermique fort soit 1) au mélange protéique au cours de la microcoagulation (ce qui rend le procédé plus tolérant vis à vis de variations de température), soit 2) au substitut de matière grasse une fois préparé (en vue de sa stabilisation par pasteurisation ou stérilisation, soit encore 3) à l'aliment allégé dans lequel le substitut a été incorporé (également en vue d'une pasteurisation ou stérilisation).

D'une manière générale, l'utilisation des compositions protéiques de l'invention permet de réduire la teneur en lipides d'un aliment favorisant ainsi le suivi d'un régime.

L'onctuosité des compositions de l'invention est également avantageusement mise à profit dans les domaines cosmétiques et pharmaceutiques, notamment pour la préparation de bases hydratantes.

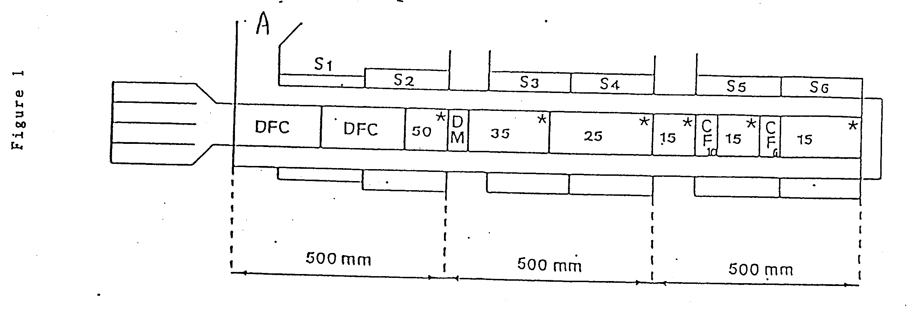

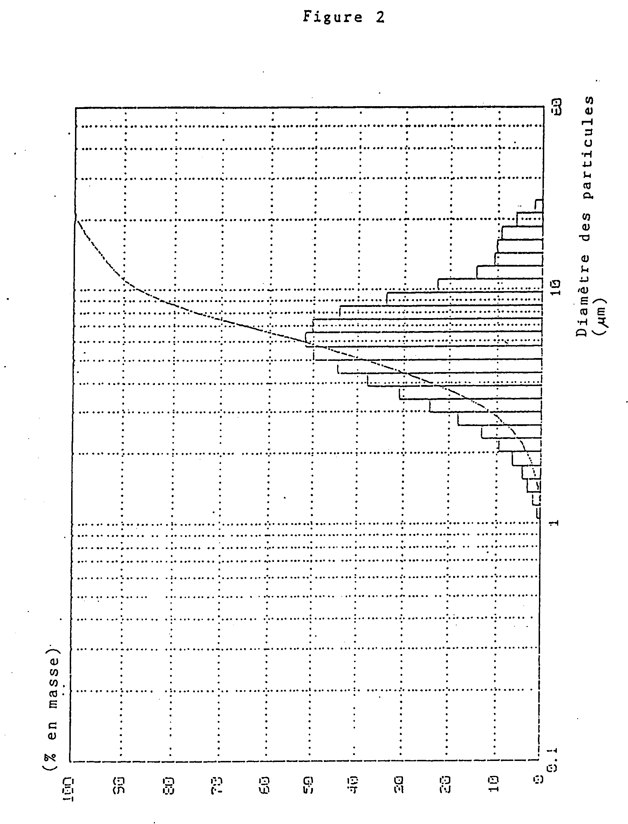

On rapporte dans les exemples qui suivent d'autres caractéristiques et avantages de l'invention. Dans ces exemples, il est fait référence aux figures 1 et 2 qui représentent :
- la figure 1, une vue schématique d'un profil de vis d'un appareil cuiseur-extrudeur utilisé pour obtenir les compositions de l'invention, et
- la figure 2, la distribution des tailles de particules d'une composition protéique de l'invention.

**Matières premières :**

. caséinate de calcium (Union Laitière Normande),
. isolat protéique de lactosérum : (Albuvir 86, Union Laitière normande, 50890 Condé-sur-Vire) (85 % de protéine par rapport au poids de matière sèche) préparé par chromatographie d'échange d'anions sur colonne de résine Sphérosil QMA.

**Préparation de mélanges caséinate de calcium-isolat protéique de lactosérum :**

les deux ingrédients, chacun sous forme pulvérulente, sont mélangés pendant une durée suffisante pour obtenir des mélanges homogènes. Le mélange final contient pour 100 g de protéines : 50 à 60 g de protéines provenant de caséinate et 50 à 40 g de protéines provenant de l'isolat protéique de lactosérum. Le pH d'une dispersion aqueuse de ce mélange portée à 20 % de protéine est de l'ordre de 6,6 à 6,8.

**Traitement de cuisson-extrusion des mélanges caséinate de calcium-isolat protéique de lactosérum :**

un cuiseur-extrudeur, de préférence un appareil bi-vis à fourreau long (1500 mm, 6 sections) (par exemple BC 45, Clextral, 42702 Firminy, France) est utilisé pour thermocoaguler le mélange protéique.

L'un des profils de vis utilisé est représenté sur la figure 1. Le mélange pulvérulent et l'eau sont introduits en A à l'aide de doseur de poudre et d'une pompe et passent successivement dans les sections $S_1$ et $S_2$ où ils sont transportés et homogénéisés, puis dans les sections $S_3$ à $S_5$ du fourreau où le mélange subit la coagulation thermique, et dans la section $S_6$ de refroidissement.

La largeur du pas de vis des filets représentée par (∗) est indiquée en mm. DFC correspond à des doubles filets conjugués, DM à des disques malaxeurs, $CF_{10}$ ou $CF_6$ à des contre filets de fente de 10 ou 6 mm respectivement.

Le débit total de matière dans l'extrudeur est fixé à une valeur comprise entre quelques kg/h et 40 kg/h dans le cas du cuiseur-extrudeur représenté. Un régime stable est assuré.

La température du fourreau métallique d'extrusion dans les sections de chauffage est maintenue entre 80 et 120°C environ, et de préférence de 85 à 90°C, afin d'obtenir la thermocoagulation recherchée des protéines globulaires. La vitesse de rotation des vis est comprise entre 100 et 600 t/min, afin d'engendrer les forces de cisaillement nécessaires à la fragmentation des protéines coagulées et obtenir des microparticules de taille n'excédant pas 20 μm. Lorsqu'on opère à des vitesses de rotation de vis élevées, un dispositif de dégazage est souhaitable pour éviter la formation de mousse.

**Caractéristiques des compositions obtenues.**

Les compositions protéiques ont un pH de 6 à 7,5 environ. L'indice de solubilité de l'azote (exprimé en g d'azote soluble pour 100 g d'azote total) est compris entre 70 et 80 %. Leur texture est toujours lisse et onctueuse, et plus ou moins ferme et tartinable selon la teneur en protéines. Cette texture est maintenue après entreposage congelé et décongélation.

Sur la figure 2, on a représenté une courbe de distribution des tailles des particules d'une composition protéique de pH 6,7 à 50/50 p/p caséinate de calcium-isolat protéique de lactosérum, de teneur totale en protéines de 20 % (p/p du produit), obtenue en chauffant le fourreau à 85°C (vitesse de rotation de vis : 100 t/min), débit : 20 kg/h).

On rapporte ci-après des exemples de substituts de matière grasse et de préparation pour base cosmétique réalisés avec des compositions de l'invention.

Exemple 1 : sauce "allégée" au fromage frais pour salades :

```
        Formulation :              Quantités en g pour
                                   100 g de produit fini


  . Fromage blanc
    (par ex. BioR de Danone)              67
  . composition protéique de
    l'invention à base de concentré protéique
    de lactosérum et de caséinate         33


  . Basilic, oignon, ail, sel, poivre     0,1
```

Caractéristiques du produit fini :

pH : 5,3
Teneur en lipides      1,6
Teneur en protéines    9,7
Teneur en glucides     1,9

Exemple 2 : mayonnaise

| Formulation | Quantités en g pour 100 g de produit fini |
|---|---|
| . Huile d'olive | 36,5 |
| . Composition protéique de l'invention à base d'isolat protéique de soja et de caséinate | 55 |
| . Jus de citron | 8,5 |
| . Basilic, oignon, ail, sel, poivre | 0,1 |

Caractéristiques du produit fini :

pH : 5,3
Teneur en lipides        36,7
Teneur en protéines      11,0
Teneur en glucides        0

Exemple 3 : mousse au chocolat

| Formulation : | Quantités en g pour 100 g de produit fini |
|---|---|
| . Chocolat noir | 24 |
| . Composition de l'invention à base d'isolat protéique de lactosérum et de caséinate | 45 |
| . Lait écrémé liquide | 8 |
| . Sucre | 7 |
| . Blanc d'oeuf | 16 |

Caractéristiques du produit fini :

pH : 6,4
Teneur en lipides        8,9
Teneur en protéines      11,7
Teneur en glucides       22,1

Exemple 4 : glace au chocolat

Formulation :                                    Quantités en g pour
                                                 100 g de produit fini


. Chocolat noir                                           22
. Lait écrémé liquide                                     22
. Composition protéique de l'invention
  à base d'isolat protéique de
  lactosérum et de caséinate                              56
. Aspartame                                             traces



Caractéristiques du produit fini :

pH : 6,5
Teneur en lipides      7,8
Teneur en protéines    12,9
Teneur en glucides     13,9

Exemple 5 : base hydratante pour crème cosmétique.


Formulation :                                    Quantités en g pour
                                                 100 g de produit fini


. glycérol                                               10
. phospholipides végétaux                                 2
. extrait xanthique                                      0,5



. composition protéique de
  l'invention à base d'isolat protéique
  de lactosérum et de caséinate                          20
. eau                                  qsp pour          100



**Revendications**

1/ Composition protéique, caractérisée en ce qu'elle est à base de caséinates et de microparticules coagulées de protéines globulaires.

2/ Composition selon la revendication 1, caractérisée en ce qu'elle renferme 30 à 60 %, de préférence 40 à 50 % en poids de protéines globulaires.

3/ Composition selon la revendication 1 ou 2, caractérisée en ce qu'elle présente un pH proche de la neutralité, en particulier de l'ordre de 6 à 7,5.

**4/** Composition selon l'une quelconque des revendications 1 à 3, caractérisée en ce que les microparticules de protéines globulaires coagulées sont dispersées dans une matrice de caséinate.

**5/** Composition selon l'une quelconque des revendications précédentes, caractérisée en ce qu'elle est formée de microparticules dont la taille n'excède pas 20 μm environ.

**6/** Composition selon l'une quelconque des revendications précédentes, caractérisée en ce que les protéines globulaires sont choisies parmi les protéines de lactosérum, les protéines de blanc d'oeuf, de soja ou de sérum sanguin, les protéines de lactosérum se présentant sous forme de concentrés ou d'isolats protéiques.

**7/** Composition protéique telle qu'obtenue par microcoagulation d'un mélange essentiellement formé de protéines globulaires et de caséinates, en appliquant un traitement thermique et des forces de cisaillement de manière à obtenir une dimension de microparticules d'au plus 20 μm environ.

**8/** Composition protéique selon l'une quelconque des revendications 1 à 7, caractérisée en ce qu'elle comporte en outre des composés inhibiteurs d'agrégation protéique ou des agents émulsifiants.

**9/** Composition protéique selon la revendication 8, caractérisée en ce que les inhibiteurs d'agrégation comprennent des polysaccharides chargés comme le xanthane, les pectines, les carrhagénates, ou les alginates, des maltodextrines, des polysaccharides neutres comme les amidons solubles, hydrolysés ou modifiés, des mono ou disaccharides et que les agents émulsifiants sont constitués par le stearoyl lactylate de sodium ou de calcium.

**10/** Procédé d'obtention d'une composition selon l'une quelconque des revendications 1 à 9, caractérisé en ce que

- on mélange les ingrédients de départ, sous forme pulvérulente, en utilisant de préférence 40 à 50 % en poids de protéine globulaire, et
- on soumet le mélange obtenu, auquel on ajoute de l'eau ou une solution diluée acide ou basique, et éventuellement des composés inhibiteurs d'agrégation ou des agents émulsifiants, tels que définis dans la revendication 9 à un traitement de cuisson-extrusion, à une température de l'ordre de 80 à 120°C, de préférence d'environ 85 à 90°C, en appliquant des forces de cisaillement permettant d'obtenir une fragmentation des protéines coagulées, et des microparticules dont la taille n'excède pas 20 μm environ.

**11/** Produits allégés, caractérisés en ce qu'ils renferment en combinaison un produit alimentaire et une composition protéique selon l'une quelconque des revendications 1 à 9.

**12/** Produits allégés selon la revendication 11, caractérisés en ce que les produits alimentaires sont des produits laitiers tels que laits, fromages frais, fromages, beurres, pâtes à tartiner, desserts, crèmes, crèmes glacées, crèmes pour nappage, yaourts.

**13/** Produits allégés selon la revendication 11 caractérisés en ce que les produits alimentaires sont du type de ceux de la revendication 12 mais à base de soja.

**14/** Produits allégés selon la revendication 11, caractérisés en ce qu'il s'agit de produits à base d'huile tels que sauces pour salades, mayonnaises, ou margarines.

**15/** Produits allégés selon la revendication 11, caractérisés en ce qu'il s'agit de pâtisseries ou de pâtes à tartiner non laitières.

**16/** Produits allégés selon la revendication 11, caractérisés en ce qu'il s'agit de charcuteries, notamment de préparations carnées émulsifiées.

**17/** Produits allégés selon la revendication 11, caractérisés en ce qu'il s'agit de plats cuisinés apertisés, congelés ou conservés réfrigérés.

**18/** Application des compositions protéiques selon l'une quelconque des revendications 1 à 9, pour l'élaboration de bases hydratantes pour la cosmétologie ou la pharmacie.

Figure 1

Figure 2